# EUROPEAN PATENT SPECIFICATION

(11) **EP 3 383 460 B1**
(45) Date of publication and mention of the grant of the patent: **15.07.2020**
(21) Application number: 16802118.6
(22) Date of filing: 30.11.2016
(51) Int. Cl.: A61M 15/06, A61M 11/04, A24F 47/00

(54) **NON-COMBUSTIBLE SMOKING DEVICE AND ELEMENTS THEREOF**
NICHTBRENNBARE RAUCHVORRICHTUNG UND ELEMENTE DAVON
DISPOSITIF À FUMER NON COMBUSTIBLE ET ÉLÉMENTS ASSOCIÉS

(30) Priority: 30.11.2015 US 201562260761 P
(43) Date of publication of application: 10.10.2018
(73) Proprietor: Philip Morris Products S.A., 2000 Neuchâtel (CH)
(72) Inventor: KARLES, Georgios D, Richmond, Virginia 23233 (US); LI, San, Midlothian, Virginia 23114 (US); RAGLAND, Ben, Providence Force, Virginia 23140 (US)
(74) Representative: Millburn, Julie Elizabeth
(86) International application number: PCT/EP2016/079344
(87) International publication number: WO 2017/093358

(56) References cited:
- WO-A1-2014/116974
- WO-A1-2015/046385
- CA-A1- 2 925 645
- US-A- 5 505 214
- US-A1- 2008 092 912
- US-A1- 2014 060 554
- US-A1- 2014 166 029
- US-A1- 2014 202 472
- US-A1- 2014 366 898

## Description

The present invention relates generally to a non-combustible smoking device.

Electronic vaping devices are used to vaporize a pre-vapor formulation into a vapor. These electronic vaping devices may be referred to as e-vaping devices. E-vaping devices include a heater, which vaporizes the pre-vapor formulation to produce the vapor. The e-vaping device may include several e-vaping elements including a power supply, a cartridge or e-vaping tank including the heater and a reservoir capable of holding the pre-vapor formulation.

US 2014/0366898 discloses a device for generating an inhalable aerosol comprising a cartridge comprising a first vaporizable material; a first compartment that contains the first vaporizable material; a first heating element; a second vaporizable material; and a second compartment that contains the second vaporizable material.

WO 2014/116974 discloses an e-cigarette comprising: a cartomizer and a battery; wherein the cartomizer comprises a glycol and water solution and an insert configured to deliver a flavor to an aerosol created by the cartomizer.

US 2014/166029 discloses an electronic cigarette comprising: a battery portion that provides power to the electronic cigarette; a cartomizer coupled with the battery portion that generates a mist; and a flavor enhancement mechanism coupled with the cartomizer.

US 2008/092912 discloses a tobacco-containing, electrically-powered smoking article comprising: an outer housing having a mouth-end and an end distal to the mouth-end, wherein the mouth-end comprises an opening adapted for egress of an aerosol generated within the smoking article and the distal end comprises an opening adapted for intake of air into the smoking article; an electrical power source within the outer housing and operatively positioned downstream of the opening in the distal end of the outer housing such that air entering the smoking article passes the electrical power source; a first electrical resistance heating element within the outer housing, powered by said electrical power source, and operatively positioned for heating air drawn through the opening in the distal end of the outer housing; a tobacco material positioned within the outer housing; an aerosol-forming material positioned within the outer housing in fluid communication with said tobacco material such that air can be drawn through both the tobacco material and the aerosol-forming material; a second electrical resistance heating element within the outer housing, powered by said electrical power source, and operatively positioned for heating the aerosol-forming material and tobacco material; and a puff-actuated controller adapted for regulating current flow through at least one of said first and second electrical resistance heating elements during draw, the controller comprising a sensor adapted for sensing draw by the user on the smoking article.

At least one example embodiment of the present invention relates to a non-combustible smoking device. The non-combustible smoking device may have at least one heater that heats a pre-vapor formulation and heats a tobacco element that receives the vapor. More specifically, the non-combustible smoke device according to example embodiments exposes a vapor to a tobacco element, exposes a pre-vapor formulation to a tobacco element, or both.

At least one example embodiment discloses a non-combustible smoking element including a pre-vapor formulation reservoir element configured to contain a pre-vapor formulation material, a heating element coupled to the pre-vapor formulation reservoir element and configured to heat at least a portion of the pre-vapor formulation material into a vapor and provide the vapor to a first channel and a tobacco containing element defining at least a portion of the first channel, the tobacco containing element overlapping at least a portion of the heating element.

In an example embodiment, the tobacco containing element is an annular sleeve.

In an example embodiment, the tobacco containing element includes an inner wall and an outer wall, the inner wall being permeable and the outer wall being impermeable.

In an example embodiment, the inner wall and the outer wall contain tobacco plant material in any form.

In an example embodiment, the tobacco containing element is arranged such that the vapor is delivered to the tobacco containing element upon an action by an adult vaper of the non-combustible smoking element.

In an example embodiment, the heating element is configured to be in thermal communication with the tobacco containing element to heat at least a portion of the tobacco containing element.

In an example embodiment, the heating element is configured to heat the portion of the tobacco containing element and produce an aroma from tobacco in the tobacco containing element.

In an example embodiment, the non-combustible smoking element further includes a wick extending in a longitudinal direction from the pre-vapor formulation reservoir element.

In an example embodiment, the pre-vapor formulation reservoir element includes an outer housing configured to contain the pre-vapor formulation material, an inner tube of the outer housing defining an air inlet, the wick in communication with the pre-vapor formulation reservoir element such that the wick is configured to draw the portion of the pre-vapor formulation material to be in thermal communication with the heating element.

In an example embodiment, the heating element extends in the longitudinal direction.

In an example embodiment, the tobacco containing element is adjacent a first end of the outer housing.

In an example embodiment, the non-combustible smoking element further includes an outer wall element on the tobacco containing element.

In an example embodiment, the outer wall element includes an outer wall part and an inner wall part, the outer wall part and the tobacco containing element defining portions of a second air channel.

In an example embodiment, the outer wall element includes a cover at a first end of the inner wall part, the cover covering the first channel.

The above and other features and advantages of example embodiments will become more apparent by describing in detail, example embodiments with reference to the attached drawings. The accompanying drawings are intended to depict example embodiments and should not be interpreted to limit the intended scope of the claims. The accompanying drawings are not to be considered as drawn to scale unless explicitly noted.
FIG. 1 is a side view of a non-combustible smoking device according to an example embodiment;
FIG. 2 is a cross-sectional view of the non-combustible smoking device of FIG. 1;
FIG. 3A illustrates an example embodiment of a non-combustible smoking device including a tobacco containing section having annular sleeves;
FIG. 3B illustrates an example embodiment of a non-combustible smoking device including a tobacco containing section having annular sleeves
FIG. 3C illustrates an example embodiment of a non-combustible smoking device including a tobacco containing section having annular sleeves;
FIG. 4 illustrates an air flow pattern of the non-combustible smoking device shown in FIG. 3A;
FIG. 5 is an enlarged view of a heater of the non-combustible smoking device of FIG. 2;
FIG. 6 illustrates an example embodiment of an end of the tobacco containing section of FIG. 2;
FIG. 7 illustrates an example embodiment of an end of the tobacco containing section of FIG. 2; and
FIG. 8 illustrates an example embodiment of an end of the tobacco containing section of FIG. 2.

Some detailed example embodiments are disclosed herein. However, specific structural and functional details disclosed herein are merely representative for purposes of describing example embodiments. Example embodiments may, however, be embodied in many alternate forms and should not be construed as limited to only the embodiments set forth herein.

Accordingly, while example embodiments are capable of various modifications and alternative forms, embodiments thereof are shown by way of example in the drawings and will herein be described in detail. It should be understood, however, that there is no intent to limit example embodiments to the particular forms disclosed, but to the contrary, example embodiments are to cover all modifications, equivalents, and alternatives falling within the scope of example embodiments. Like numbers refer to like elements throughout the description of the figures.

It should be understood that when an element or layer is referred to as being "on," "connected to," "coupled to," or "covering" another element or layer, it may be directly on, connected to, coupled to, or covering the other element or layer or intervening elements or layers may be present. In contrast, when an element is referred to as being "directly on," "directly connected to," or "directly coupled to" another element or layer, there are no intervening elements or layers present. Like numbers refer to like elements throughout the specification.

It should be understood that, although the terms first, second, third, and so forth may be used herein to describe various elements, regions, layers or sections, these elements, regions, layers, or sections should not be limited by these terms. These terms are only used to distinguish one element, region, layer, or section from another element, region, layer, or section. Therefore, a first element, region, layer, or section discussed below could be termed a second element, region, layer, or section without departing from the teachings of example embodiments.

Spatially relative terms (for example, "beneath," "below," "lower," "above," "upper," and the like) may be used herein for ease of description to describe one element or feature's relationship to one or more other elements or features as illustrated in the figures. It should be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Therefore, the term "below" may encompass both an orientation of above and below. The device may be otherwise oriented (rotated 90 degrees or at other orientations) and the spatially relative descriptors used herein interpreted accordingly.

The terminology used herein is for the purpose of describing various embodiments only and is not intended to be limiting of example embodiments. As used herein, the singular forms "a," "an," and "the" are intended to include the plural forms as well, unless the context clearly indicates otherwise. It will be further understood that the terms "includes," "including," "comprises," and "comprising," when used in this specification, specify the presence of stated features, integers, steps, operations, or elements, but do not preclude the presence or addition of one or more other features, integers, steps, operations, elements, or groups thereof.

Example embodiments are described herein with reference to cross-sectional illustrations that are schematic illustrations of idealized embodiments (and intermediate structures) of example embodiments. As such, variations from the shapes of the illustrations as a result, for example, of manufacturing techniques or tolerances, are to be expected. Therefore, example embodiments should not be construed as limited to the shapes of regions illustrated herein but are to include deviations in shapes that result, for example, from manufacturing. Therefore, the regions illustrated in the figures are schematic in nature and their shapes are not intended to illustrate the actual shape of a region of a device and are not intended to limit the scope of example embodiments.

Unless otherwise defined, all terms (including technical and scientific terms) used herein have the same meaning as commonly understood by one of ordinary skill in the art to which example embodiments belong. It will be further understood that terms, including those defined in commonly used dictionaries, should be interpreted as having a meaning that is consistent with their meaning in the context of the relevant art and will not be interpreted in an idealized or overly formal sense unless expressly so defined herein.

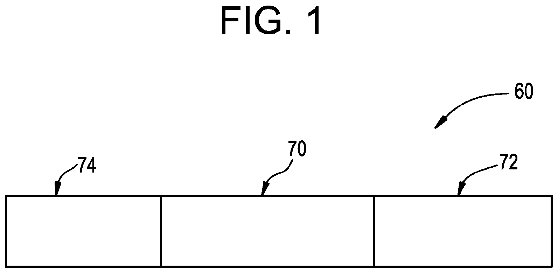

FIG. 1 illustrates a non-combustible smoking device 60 according to an example embodiment. The non-combustible smoking device 60 includes a replaceable cartridge (or first section) 70, a reusable fixture (or second section) 72 and a tobacco containing section (or third section) 74.

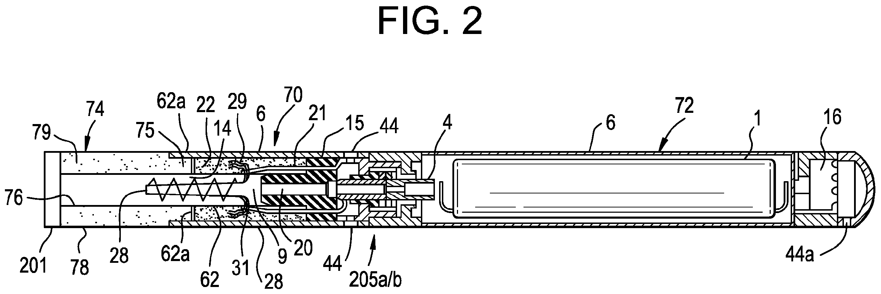

FIG. 2 is a cross-sectional view of the non-combustible smoking device of FIG. 1. As shown, the replaceable cartridge 70 and the reusable fixture 72 are coupled together at a connection 205a, 205b (for example, 205a is a male threaded connection on cartridge 70, and 205b is a female threaded connection on reusable fixture 72) or by other convenience such as at least one of a snug-fit, detent, clamp, or clasp.

The first section 70 includes an outer tube 6 (or housing) extending in a longitudinal direction and an inner tube 62 coaxially positioned within the outer tube or housing 6. The inner tube 62 defines a portion of an outer air passage (or channel) 9.

A portion 75 of the tobacco containing section 74 fits within a circumference defined by an inner portion of the outer tube 6 to create a frictional connection between the tobacco containing section 74 and the cartridge 70. Example embodiments are not limited to the frictional connection and other connections may be used. Therefore, the tobacco containing section 74 is a detachable insert.

The tobacco containing section 74 includes an inner tube 76 and an outer wall 78. The inner tube 76 of the tobacco containing section 74 defines another portion of the outer air passage 9. The outer wall 78 and the inner tube 76 define a space (annulus) therebetween.

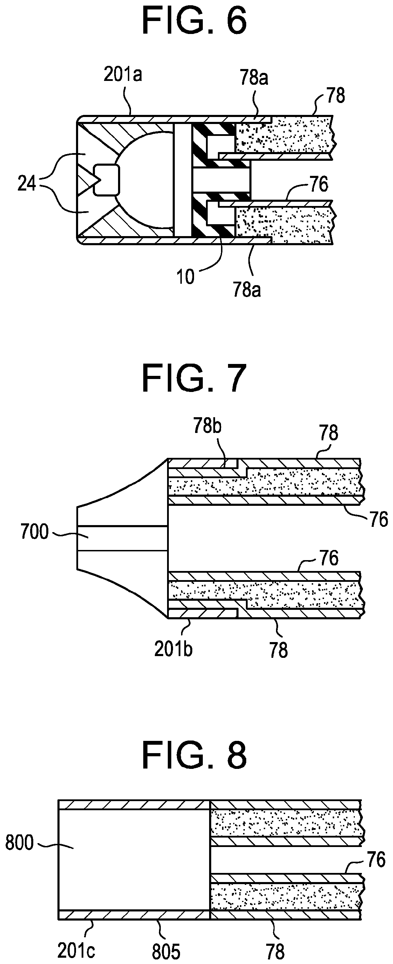

An end 201 of the tobacco containing section 74 may be a low efficiency cellulose acetate filter, a hollow acetate tube, or a plastic or wood mouthpiece. When the end 201 is a plastic or wood mouthpiece, the end 201 is shaped such that a portion of the outer wall 78 fits within a circumference of the end 201. FIGS. 6-8 illustrate example embodiments of the end 201.

Within the space between the outer wall 78 and the inner tube 76, the tobacco containing section 74 includes a tobacco element 79. The term "tobacco element" may refer to any tobacco plant material including tobacco leaf, tobacco plug, reconstituted tobacco, compressed tobacco rod, shaped, or powder, for example.

In addition, the inner tube 76 and the outer wall 78 may contain tipping paper, a tobacco plant material in any form including rolled natural or reconstituted tobacco leaf or sheet or from an annular piece made of tobacco filler or extruded tobacco in the shape of a sleeve. The inner tube 76 and the outer wall 78 may be made of the same or different materials.

In an example embodiment, the tobacco containing section 74 may be a filtered cigarette, a non-filtered cigarette, a cigarillo, a filter tipped cigar filter, a tipped cigar, an untipped cigar or an untipped cigarillo, for example. However, example embodiments are not limited thereto. If the tobacco containing section 74 is a shortened cigarette, the tobacco containing section 74 may include a filter at the end 201. In example embodiments where the tobacco insert is an untipped cigar or an untipped cigarillo, the tobacco insert does not include a filter.

The filter may be a low efficiency cellulose acetate (CA) filter. CA filter elements, such as triacetin, can be eluted into vapor. Vapor phase nicotine and other volatile elements in vapor can be reduced by a presence of tobacco.

A heater 14 extends in a longitudinal direction from the inner tube 62 into the inner tube 76 in the outer air passage 9.

The second section 72 can also include the outer tube 6 extending in a longitudinal direction. In an alternative embodiment, the outer tube 6 can be a single tube housing both the first section 70 and the second section 72 and the entire non-combustible smoking device 60 can be disposable.

The non-combustible smoking device 60 can also include a central air passage 20 defined in part by the inner tube 62 and an upstream seal 15. Moreover, the non-combustible smoking device 60 includes a pre-vapor formulation supply reservoir 22. The pre-vapor formulation supply reservoir 22 comprises a pre-vapor formulation material and optionally a pre-vapor formulation storage medium 21 operable to store the pre-vapor formulation material therein.

In an embodiment, the pre-vapor formulation supply reservoir 22 is contained in an outer annulus between the outer tube 6 and the inner tube 62. The annulus is sealed at an upstream end by the seal 15. At a downstream end, the annulus is sealed by a gasket 62a. The gasket 62a may be a ring shaped gasket.

The gasket 62a is placed on the pre-vapor formulation supply reservoir 22 to seal the pre-vapor formulation in the pre-vapor formulation supply reservoir 22 and prevent the tobacco element 79 from mixing with the pre-vapor formulation.

In an embodiment, the heater 14 is also contained in the inner tube 62 downstream of and in spaced apart relation to the portion of central air passage 20 defined by the seal 15. The heater 14 can be in the form of a wire coil, a planar body, a ceramic body, a single wire, a cage of resistive wire or any other suitable form.

A wick 28 is in communication with the pre-vapor formulation material in the pre-vapor formulation supply reservoir 22 and in communication with the heater 14 such that the wick 28 disposes pre-vapor formulation material in proximate relation to the heater 14. The wick 28 may be constructed of a fibrous and flexible material. The wick 28 may include at least one filament having a capacity to draw a pre-vapor formulation. For example, the wick 28 may comprise a bundle of filaments which may include glass (or ceramic) filaments. In another embodiment, a bundle comprising a group of windings of glass filaments, for example, three of such windings, all which arrangements are capable of drawing pre-vapor formulation via capillary action via interstitial spacing between the filaments.

A power supply 1 in the second section 72 may be operably connected to the heater 14 (as described below) to apply voltage across the heater 14. The non-combustible smoking device 60 also includes at least one air inlet 44 operable to deliver air to the central air passage 20, other portions of the inner tube 62, or both.

Moreover, the heater 14 extends in the longitudinal direction and heats the pre-vapor formulation material to a temperature sufficient to vaporize the pre-vapor formulation material and form a vapor when a negative pressure is applied to the end 201. In other embodiments, the heater 14 may be arranged in another manner such as in a direction transverse to the longitudinal direction.

The vapor then flows through the inner tube 76 and into the tobacco element 79 upon a negative pressure being applied at the end 201 of the tobacco containing section 74. The heater 14 may be a set distance from the tobacco element 79 such that the heater 14 heats the tobacco element 79 when a negative pressure is applied. For example, the heater 14 may be about 10 millimeters or less from the inner tube 76.

The heater 14 may extend into the tobacco containing portion 74 between about 5 and about 20 millimeters. The heater 14 may be arranged to produce a temperature of about 50 degrees Celsius at the end 201. Moreover, the heater 14 may heat the tobacco element 79 to a temperature between about 50 and about 200 degrees Celsius and heat the pre-vapor formulation at between about 300 and about 350 degrees Celsius.

The heater 14 warms the tobacco element 79, but does not burn the tobacco. Therefore, the warming of the tobacco element 79 may be referred to as non-combustible. Because the section 70 includes the heater 14 and the tobacco containing section 74 includes the tobacco element 79, the sections 70 and 74 may jointly be referred to as a non-combustible smoking element.

In one embodiment, the first section (the cartridge) 70 and the tobacco containing section 74 are disposable and the second section (the fixture) 72 is reusable. The sections 70, 72 can be attached by a threaded connection 205, as described above, whereby the downstream section 70 can be replaced when the pre-vapor formulation supply reservoir 22 is used up. Optionally, the first section 70 and the second section 72 are arranged to releaseably lock together when engaged.

In an embodiment, the at least one air inlet 44 includes one or two air inlets. Alternatively, there may be three, four, five or more air inlets. If there is more than one air inlet 44, the air inlets 44 are located at different locations along the non-combustible smoking device 60. For example, as shown in FIG. 2, an air inlet 44a can be positioned at the upstream end of the non-combustible smoking device 60 adjacent a sensor 16 such that the sensor 16 supplies power to the heater 14 upon sensing a negative pressure. Air inlet 44a allows a negative pressure applied at the end 201 to activate the sensor 16. The air from the air inlet 44a can then flow along the power supply 1 and to at least one of the central air passage 20 in the seal 15 and other portions of at least one of the inner tube 62 and the outer tube 6. At least one additional air inlet 44 can be located adjacent and upstream of the seal 15 or at any other desirable location. Altering the size and number of air inlets 44 can also aid in establishing the resistance to draw of the non-combustible smoking device 60.

In an embodiment, the heater 14 is arranged to communicate with the wick 28 and to heat the pre-vapor formulation material contained in the wick 28 to a temperature sufficient to vaporize the pre-vapor formulation material and form a vapor.

The heater 14 may be a wire coil surrounding the wick 28. Examples of suitable electrically resistive materials include titanium, zirconium, tantalum and metals from the platinum group. Examples of suitable metal alloys include stainless steel, nickel-, cobalt-, chromium-, aluminium- titanium- zirconium-, hafnium-, niobium-, molybdenum-, tantalum-, tungsten-, tin-, gallium-, manganese- and iron-containing alloys, and superalloys based on nickel, iron, cobalt, stainless steel. For example, the heater may be formed of nickel aluminides, a material with a layer of alumina on the surface, iron aluminides and other composite materials, the electrically resistive material may optionally be embedded in, encapsulated or coated with an insulating material or vice-versa, depending on the kinetics of energy transfer and the external physicochemical properties required. In one embodiment, the heater 14 comprises at least one material selected from the group consisting of stainless steel, copper, copper alloys, nickel-chromium alloys, superalloys and combinations thereof. In an embodiment, the heater 14 is formed of nickel-chromium alloys or iron-chromium alloys. In one embodiment, the heater 14 can be a ceramic heater having an electrically resistive layer on an outside surface thereof.

In another embodiment, the heater 14 may be constructed of an iron-aluminide (for example, FeAl or Fe.sub.3AI), such as those described in U.S. Pat. No. 5,595,706 to Sikka et al. filed Dec. 29, 1994, or nickel aluminides (for example, Ni.sub.3AI). FeAl exhibits a resistivity of approximately 180 micro-ohms, whereas stainless steel exhibits approximately 50 to 91 micro-ohms. The higher resistivity lowers current draw or load on the power supply (battery) 1.

In one embodiment, the heater 14 comprises a wire coil which at least partially surrounds the wick 28. In that embodiment, the wire may be at least one of a metal wire and the heater coil that extends partially along the length of the wick 28. The heater coil may extend fully or partially around the circumference of the wick 28. In another embodiment, the heater coil is not in contact with the wick 28.

The heater 14 heats the pre-vapor formulation in the wick 28 by thermal conduction. Alternatively, heat from the heater 14 may be conducted to the pre-vapor formulation by means of a heat conductive element or the heater 14 may transfer heat to the incoming ambient air that is drawn through the non-combustible smoking device 60 during use, which in turn heats the pre-vapor formulation by convection.

In one embodiment, the wick comprises a ceramic material or ceramic fibers. As noted above, the wick 28 is at least partially surrounded by the heater 14. Moreover, in an embodiment, the wick 28 extends through opposed openings in the inner tube 62 such that end portions 29, 31 of the wick 28 are in contact with the pre-vapor formulation supply reservoir 22.

The wick 28 may comprise a plurality or bundle of filaments. In one embodiment, the filaments may be generally aligned in a direction transverse to the longitudinal direction of the non-combustible smoking device 60, but the example embodiments are not limited to this orientation. In one embodiment, the structure of the wick 28 is formed of ceramic filaments capable of drawing the pre-vapor formulation via capillary action via interstitial spacing between the filaments to the heater 14. The wick 28 can include filaments having a cross-section which is generally cross-shaped, clover-shaped, Y-shaped or in any other suitable shape.

The wick 28 includes any suitable material or combination of materials. Examples of suitable materials are glass filaments, surface treated steel wires, and ceramic or graphite based materials. Moreover, the wick 28 may have any suitable capillarity to accommodate pre-vapor formulations having different physical properties such as density, viscosity, surface tension and vapor pressure. The capillary properties of the wick 28, combined with the properties of the pre-vapor formulation, ensure that the wick 28 is always wet in the area of the heater 14 to avoid overheating of the heater 14.

Instead of using a wick, the heater can be a porous material of sufficient capillarity and which incorporates a resistance heater formed of a material having a high electrical resistance capable of generating heat quickly.

In one embodiment, the wick 28 and the pre-vapor formulation storage medium 21 of the pre-vapor formulation supply reservoir 22 are constructed from an alumina ceramic. In another embodiment, the wick 28 includes glass fibers and the pre-vapor formulation storage medium 21 includes a cellulosic material or polyethylene terephthalate.

In an embodiment, the power supply 1 may include a battery arranged in the non-combustible smoking device 60 such that the anode is downstream of the cathode. An anode connector 4 contacts the downstream end of the battery. The heater 14 is connected to the battery by two spaced apart electrical leads.

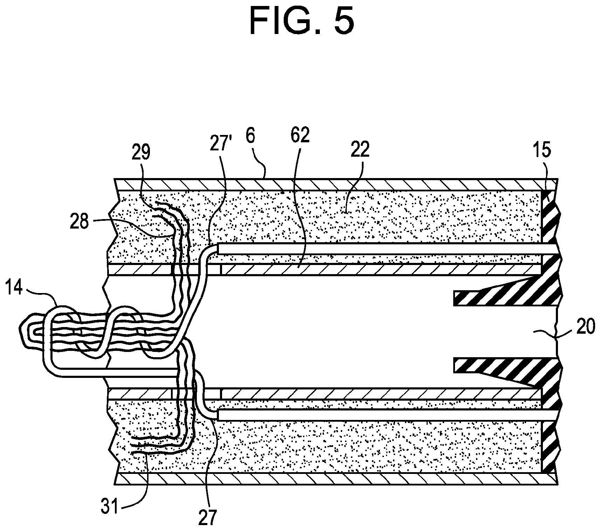

The connection between the uncoiled, end portions 27, 27' (see FIG. 5) of the heater 14 and the electrical leads are highly conductive and temperature resistant while the heater 14 is highly resistive so that heat generation occurs primarily along the heater 14 and not at the contacts. The end portion 27 is connected to the anode connector 4 and the end portion 27' is connected to the cathode through the outer tube 6.

The battery may be a Lithium-ion battery or one of its variants, for example a Lithium-ion polymer battery. Alternatively, the battery may be a Nickel-metal hydride battery, a Nickel cadmium battery, a Lithium-manganese battery, a Lithium-cobalt battery or a fuel cell. In that case, the non-combustible smoking device 60 is usable until the energy in the power supply is depleted. Alternatively, the power supply 1 may be rechargeable and include circuitry allowing the battery to be chargeable by an external charging device. In that case, the circuitry, when charged, provides power for a desired (or alternatively a pre-determined) number of applications of negative pressure, after which the circuitry must be re-connected to an external charging device.

The non-combustible smoking device 60 also includes control circuitry including the sensor 16. The sensor 16 is operable to sense an air pressure drop and initiate application of voltage from the power supply 1 to the heater 14.

The air inlet 44a is located adjacent the sensor 16, such that the sensor 16 senses air flow indicative of a negative pressure and activates the power supply 1.

A control circuit is integrated with the sensor 16 and supplies power to the heater 14 responsive to the sensor 16, for example, with a maximum, time-period limiter.

Alternatively, the control circuitry may include a manually operable switch to initiate an application of negative pressure. The time-period of the electric current supply to the heater may be pre-set depending on the amount of pre-vapor formulation desired to be vaporized. The control circuitry may be programmable for this purpose. Alternatively, the circuitry may supply power to the heater as long as the sensor 16 detects a pressure drop.

When activated, the heater 14 heats a portion of the wick 28 surrounded by the heater for less than about 10 seconds, more preferably less than about 7 seconds. Therefore, the power cycle can range in period from about 2 seconds to about 10 seconds (for example, about 3 seconds to about 9 seconds, about 4 seconds to about 8 seconds or about 5 seconds to about 7 seconds).

In an embodiment, the pre-vapor formulation supply reservoir 22 includes the pre-vapor formulation storage medium 21 containing pre-vapor formulation material. In FIG. 2, the pre-vapor formulation supply reservoir 22 is contained in an outer annulus between inner tube 62 and outer tube 6 and between gasket 10 and the seal 15. Therefore, the pre-vapor formulation supply reservoir 22 at least partially surrounds the central air passage 20 and the heater 14 and the wick 28 extend between portions of the pre-vapor formulation supply reservoir 22.

The pre-vapor formulation storage medium 21 may be a fibrous material comprising cotton, polyethylene, polyester, rayon and combinations thereof. The fibers may have a diameter ranging in size from about 6 microns to about 15 microns (for example, about 8 microns to about 12 microns or about 9 microns to about 11 microns). The pre-vapor formulation storage medium 21 may be a sintered, porous or foamed material. Also, the fibers may be sized to be irrespirable and can have a cross-section which has a y shape, cross shape, clover shape or any other suitable shape.

In another example embodiment, the pre-vapor formulation storage medium 21 may be a tobacco filler or tobacco slurry.

Also, the pre-vapor formulation material has a boiling point suitable for use in the non-combustible smoking device 60. If the boiling point is too high, the heater 14 will not be able to vaporize the pre-vapor formulation in the wick 28. However, if the boiling point is too low, the pre-vapor formulation may vaporize without the heater 14 being activated.

A pre-vapor formulation is a material or combination of materials that may be transformed into a vapor. For example, the pre-vapor formulation may be at least one of a liquid, solid, and gel formulation including, but not limited to, water, beads, solvents, active ingredients, ethanol, plant extracts, natural or artificial flavors, vapor formers such as glycerine and propylene glycol, and combinations thereof.

The pre-vapor formulation may include a tobacco element including volatile tobacco flavor compounds which are released upon heating. When the tobacco element is in the pre-vapor formulation the physical integrity of the tobacco element is preserved. For example, the tobacco element may be between about 2% and about 30% by weight in the pre-vapor formulation.

For example, the tobacco element may be in the form of a sheet or shreds and is added after the pre-vapor formulation is added to the pre-vapor formulation storage medium 21.

In operation, with non-combustible smoking device 60 in an assembled configuration, a negative pressure may be applied on the end 201. This may cause an internal pressure drop inside non-combustible smoking device 60 that may cause an inlet air flow to enter device 60 via air inlets 44, 44a. The internal pressure drop may also cause an internal pressure drop within section 72 as air is drawn through air inlet 44a (via an air flow path traveling through section 72). The internal pressure drop formed in section 72 may be sensed by sensor 16. The sensor 16 may then operate to close an electrical circuit that includes the power supply 1. In turn, electrical leads carry an electrical current to heater 14 in order to energize the heater 14. The energized heater 14 in turn heats and vaporizes the pre-vapor formulation material that is drawn toward the heater 14 via the wick 28.

The pre-vapor formulation material is transferred from one or both of the pre-vapor formulation supply reservoir 22 and the pre-vapor formulation storage medium 21 in proximity of the heater 14 by capillary action in the wick 28. In one embodiment, the wick 28 has a first end portion 29 and a second opposite end portion 31. The first end portion 29 and the second end portion 31 extend into opposite sides of the pre-vapor formulation storage medium 21 for contact with pre-vapor formulation material contained therein. The heater 14 at least partially surrounds a central portion of the wick 28 such that when the heater 14 is activated, the pre-vapor formulation in the central portion of the wick 28 is vaporized by the heater 14 to vaporize the pre-vapor formulation material and form vapor. Due to a negative pressure being applied, the vapor flows from the heater 14, through the tobacco element 79 and out of the end 201.

The vapor may elute tobacco elements into the flow stream. Some thermal reactions may also be present between the vapor and the tobacco element.

One advantage of an embodiment is that the pre-vapor formulation material in the pre-vapor formulation supply reservoir 22 is protected from oxygen (because oxygen cannot generally enter the pre-vapor formulation storage portion via the wick) so that the risk of degradation of the pre-vapor formulation material is significantly reduced. Moreover, in some embodiments in which the outer tube 6 is not clear, the pre-vapor formulation supply reservoir 22 is protected from light so that the risk of degradation of the pre-vapor formulation material is significantly reduced. Therefore, a high level of shelf-life and cleanliness can be maintained.

The arrangement of the section 70 is not limited to the embodiment shown in FIG. 2 and may include other modifications such as those described in U.S. Patent Application. No. 14/572,360.

One or both of the outer tube 6 and the inner tube 62 may be formed of any suitable material or combination of materials. Examples of suitable materials include metals, alloys, plastics or composite materials containing one or more of those materials, or thermoplastics that are suitable for food or pharmaceutical applications, for example polypropylene, polyetheretherketone (PEEK), ceramic, and polyethylene. In one embodiment, the material is light and non-brittle.

While FIG. 2 illustrates the tobacco containing section 74 having a singular annular sleeve, example embodiments are not limited thereto.

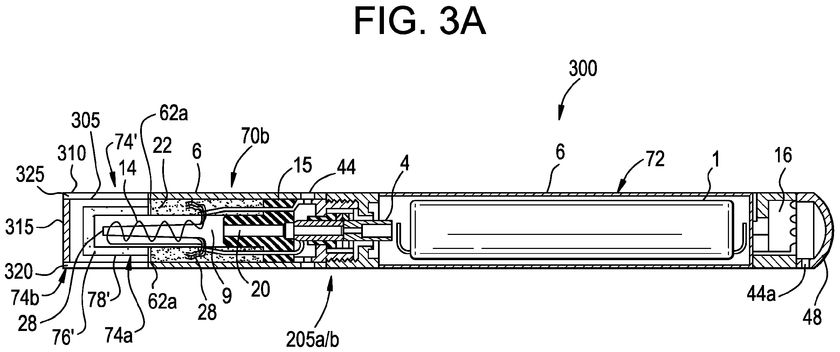

FIG. 3A illustrates an example embodiment of a non-combustible smoking device including a tobacco containing section 74' having annular sleeves 74a and 74b. A non-combustible smoking device 300 is similar to the non-combustible smoking device 60. Therefore, for the sake of brevity, only the differences will be described.

In FIG. 3A, a tobacco containing section 74' includes annular sleeves 74a and 74b.

The annular sleeve 74a includes an inner tube 76' and an outer wall 78'. The inner tube 76' defines another portion of the outer air passage 9. The outer wall 78' and the inner tube 76' define a space (annulus) therebetween. The outer wall 78' and the inner tube 76' may be made of the same materials of the outer wall 78 and inner tube 76, respectively.

Within the space between the outer wall 78' and the inner tube 76' is the tobacco element 79.

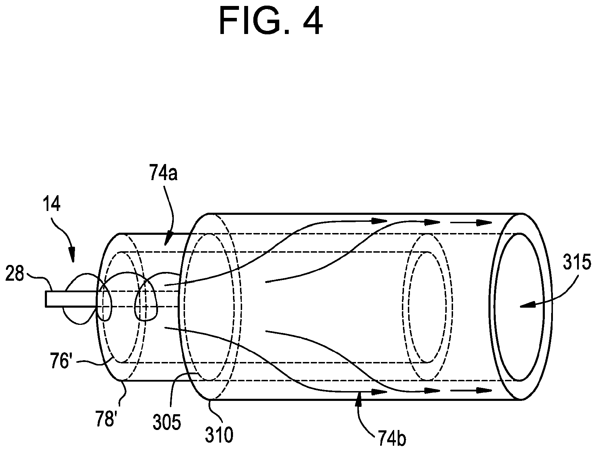

The annular sleeve 74b includes an inner tube 305 and an outer wall 310. As shown in FIG. 3A, the annular sleeve 74b encompasses the annular sleeve 74a. The inner tube 305 is permeable and the outer wall 310 is impermeable. An end 315 of the annular sleeve 74b is closed to air flow. The end 315 may be made of any material that acts as a plug to block airflow such as a plastic (for example, polyethylene) or a metal. Therefore, air flows from the air passage 9, through the annular sleeve 74a through the inner tube 305 and into air channels 320, 325 upon applying a negative pressure to the tobacco containing section 74", as shown in FIG. 4.

The inner tube 305 is a permeable material such as a membrane, mesh, perforated plastic or paper. The inner tube 305 is made of a material that maintains the structural integrity of the annular sleeve 74b. The outer wall 310 is an impermeable material such as a plastic.

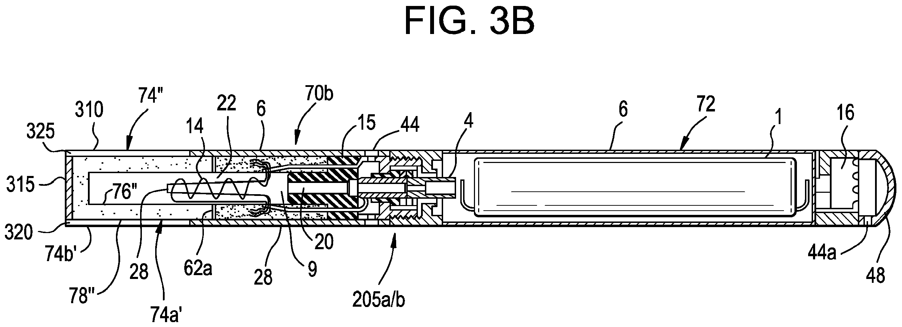

FIG. 3B illustrates another example embodiment of a non-combustible smoking device including a tobacco containing section 74" having annular sleeves 74a' and 74b'.

The tobacco containing section 74" is similar to the tobacco containing section 74'. Therefore, only the differences will be described.

In FIG. 3B, an annular sleeve 74b' does not include the inner tube 305. Instead, an outer wall 78" of the annular sleeve 74a' is also part of the annular sleeve 74b'. With an inner tube 76", the outer wall 78" and the inner tube 76" define a space (annulus) therebetween. Within the space between the outer wall 78" and the inner tube 76" is the tobacco element 79.

As shown in FIG. 3B, the outer wall 78" and the inner tube 76" extend to the end 315. The outer wall 78" and the inner tube 76" may be made of the same materials as the outer wall 78' and the inner tube 76", respectively.

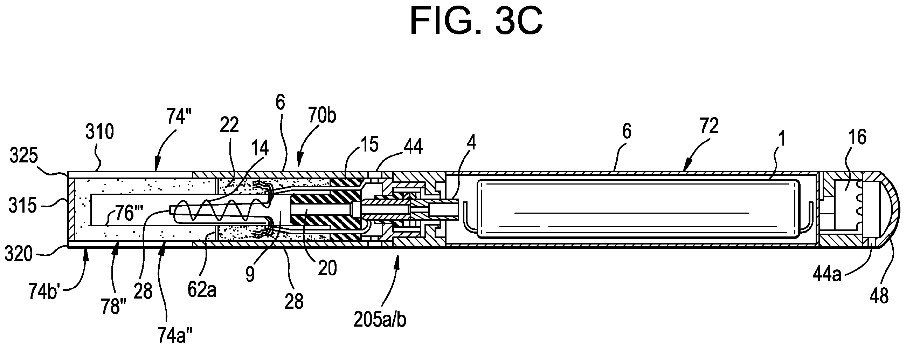

FIG. 3C illustrates another example embodiment of a non-combustible smoking device including a tobacco containing section 74'".

The tobacco containing section 74'" is similar to the tobacco containing section 74". Therefore, only the differences will be described.

In FIG. 3B, an inner tube 76'" of an annular sleeve 74a" is closed off before the end 315. A space is then defined between the end 315 and the inner tube 76"'. Tobacco element 79 is also between the end 315 and the inner tube 76'".

The non-combustible smoking devices according to example embodiments are effective in heating the tobacco and distilling and eluting tobacco specific flavors because of their flow pattern and proximity of the tobacco element to the heater 14 (vapor forming area). The perpendicular flow, shown in FIG. 4, of the vapor from the heater 14 to the tobacco element and the closeness of the tobacco to the heater 14 allow for effective heating of the tobacco and subsequent distillation and elution of volatile tobacco flavors.

While example embodiments illustrate that vapor can exit the non-combustible smoking device in an annular fashion, it should be understood that the vapor may exit in a concentric fashion.

FIG. 6 illustrates an example embodiment of an end of the tobacco containing section 74 being a plastic mouthpiece. As shown in FIG. 6, an end 201a has at least two off-axis, diverging outlets 24. The end 201a is in fluid communication with the central air passage 9, which extends through a gasket 10. The gasket 10 is at a downstream end of the tobacco containing section 74 so as to prevent leakage of the tobacco material into the end 201.

A portion of the outer wall 78a fits within a circumference of the end 201a.

Due to a negative pressure being applied to the tobacco containing section 74, the vapor flows from the heater 14, through the tobacco containing section 74 and out of the end 201a.

FIG. 7 illustrates an example embodiment of an end of the tobacco containing section 74.

An end 201b fits over a portion of the outer wall 78b. A negative pressure may be applied on the end 201b. Due to the negative pressure, the vapor flows from the heater 14, out of the tobacco containing section 74 through an air passage 700.

FIG. 8 illustrates an example embodiment of an end of the tobacco containing section 74.

An end 201c includes a filter 800. In example embodiments where the tobacco insert is an untipped cigar or an untipped cigarillo, the tobacco insert does not include a filter.

Tipping paper 805 may overlap the filter 800. Tipping paper may also be used as the wall 78. Therefore, the tipping paper 1255 provides stiffness to the tobacco containing section 74, permitting easier insertion to the cartridge 70. An aluminum foil may also be used to contain the tobacco element, with or without additional tipping paper.

In the example shown in FIG. 8, the filter 800 may be a cellulose acetate (CA) filter. CA filter elements, such as triacetin, can be eluted into vapor. Vapor phase nicotine and other volatile elements in vapor can be reduced by a presence of tobacco.

When a negative pressure is applied to the tobacco containing section 74, the vapor flows from the heater 14, through the tobacco containing section 74 and out of the filter 800.

Example embodiments provide a non-combustible smoking device having a heater that heats a pre-vapor formulation and may provide heat to a tobacco element. More specifically, the non-combustible smoke device according to example embodiments exposes a vapor to a tobacco element, exposes a pre-vapor formulation to a tobacco element, or both. When the tobacco element is in the pre-vapor formulation the physical integrity of the tobacco element is preserved.

In other example embodiments, a non-combustible smoke device can be a pod device or tank device that exposes a vapor to a tobacco element, exposes a pre-vapor formulation to a tobacco element, or both.

While a single heater is described with reference to FIGS. 1-8, example embodiments may include a multiple heater non-combustible smoking device. A first heater may be the heater 14 to vaporize the pre-vapor formulation and a second heater may be used to heat the tobacco element. The second heater may penetrate the tobacco element.

In other example embodiments, a non-combustible smoking device includes more than two heaters.

Example embodiments having therefore been described, it will be obvious that the same may be varied in many ways. Such variations are not to be regarded as a departure from the intended scope of example embodiments, and all such modifications as would be obvious to one skilled in the art are intended to be included within the scope of the following claims.

## Claims

1. A non-combustible tobacco smoking element (60) comprising:
a pre-vapor formulation reservoir element (22) configured to contain a pre-vapor formulation material;
a heating element (14) coupled to the pre-vapor formulation reservoir element and configured to heat at least a portion of the pre-vapor formulation material into a vapor and provide the vapor to a first channel; and
a tobacco containing element (79) defining at least a portion of the first channel, the tobacco containing element (79) overlapping at least a portion of the heating element (14), wherein the tobacco containing element (79) includes an inner wall (305) and an outer wall (310), the inner wall (305) being permeable and the outer wall (310) being impermeable, wherein the inner wall (305) and the outer wall (310) contain tobacco plant material in any form.

2. The non-combustible tobacco smoking element (60) of claim 1, wherein the tobacco containing element (79) is an annular sleeve.

3. The non-combustible tobacco smoking element (60) of any preceding claim, wherein the heating element (14) is configured to be in thermal communication with the tobacco containing element (79) to heat at least a portion of the tobacco containing element.

4. The non-combustible tobacco smoking element (60) of claim 3, wherein the heating element (14) is configured to heat the portion of the tobacco containing element (79) and produce an aroma from tobacco in the tobacco containing element.

5. The non-combustible tobacco smoking element (60) of any preceding claim, further comprising:
a wick (28) extending in a longitudinal direction from the pre-vapor formulation reservoir element (22).

6. The non-combustible tobacco smoking element (60) of claim 5, wherein the pre-vapor formulation reservoir element (22) includes,
an outer housing configured to contain the pre-vapor formulation material, an inner tube of the outer housing defining an air inlet, the wick (28) in communication with the pre-vapor formulation reservoir element (22) such that the wick (28) is configured to draw the portion of the pre-vapor formulation material to be in thermal communication with the heating element (14).

7. The non-combustible tobacco smoking element (60) of claim 6, wherein the tobacco containing element (79) is adjacent a first end of the outer housing.

8. The non-combustible tobacco smoking element (60) of claim 5, 6, or 7 wherein the heating element extends in the longitudinal direction.

9. The non-combustible tobacco smoking element (60) of any preceding claim, further comprising:
an outer wall element on the tobacco containing element (79).

10. The non-combustible tobacco smoking element (60) of claim 9, wherein the outer wall element includes,
an outer wall part; and
an inner wall part, the outer wall part and the tobacco containing element (79)defining portions of a second air channel.

11. The non-combustible tobacco smoking element (60) of claim 10, wherein the outer wall element includes,
a cover at a first end of the inner wall part, the cover covering the first channel.

## Patentansprüche

1. Nicht brennbares Tabakrauchelement (60), aufweisend:
ein Vordampfformulierungsvorratsbehälterelement (22), das derart ausgelegt ist, dass es ein Vordampfformulierungsmaterial enthält;
ein Heizelement (14), das mit dem Vordampfformulierungsvorratsbehälterelement gekoppelt und ausgelegt ist, mindestens einen Teil des Vordampfformulierungsmaterials zu Dampf zu erwärmen und den Dampf an einen ersten Kanal bereitzustellen; und
ein tabakhaltiges Element (79), das mindestens einen Teil des ersten Kanals definiert, wobei das tabakhaltige Element (79) mindestens einen Teil des Heizelements (14) überlappt, wobei das tabakhaltige Element (79) eine Innenwand (305) und eine Außenwand (310) aufweist, die Innenwand (305) durchlässig und die Außenwand (310) undurchlässig ist, wobei die Innenwand (305) und die Außenwand (310) Tabakpflanzenmaterial in irgendeiner Form enthalten.

2. Nicht brennbares Tabakrauchelement (60) nach Anspruch 1, wobei das tabakhaltige Element (79) eine ringförmige Hülse ist.

3. Nicht brennbares Tabakabrauchelement (60) nach einem der vorstehenden Ansprüche, wobei das Heizelement (14) derart ausgelegt ist, dass es in thermischer Verbindung mit dem tabakhaltigen Element (79) steht, um mindestens einen Teil des tabakhaltigen Elements zu erwärmen.

4. Nicht brennbares Tabakrauchelement (60) nach Anspruch 3, wobei das Heizelement (14) ausgelegt ist, den Abschnitt des tabakhaltigen Elements (79) zu erwärmen und ein Aroma von Tabak in dem tabakhaltigen Element zu erzeugen.

5. Nicht brennbares Tabakrauchelement (60) nach einem der vorstehenden Ansprüche, weiter aufweisend:
einen Docht (28), der sich in einer Längsrichtung von dem Vordampfformulierungsvorratsbehälterelement (22) erstreckt.

6. Nicht brennbares Tabakrauchelement (60) nach Anspruch 5, wobei das Vordampfformulierungsvorratsbehälterelement (22) einschließt,
ein Außengehäuse, das derart ausgelegt ist, dass es das Vordampfformulierungsmaterial enthält, wobei ein Innenrohr des Außengehäuses einen Lufteinlass definiert, der Docht (28) in Verbindung mit dem Vordampfformulierungsvorratsbehälterelement (22) steht, sodass der Docht (28) ausgelegt ist, den Teil des Vordampfformulierungsmaterials zu ziehen, der in thermischer Verbindung mit dem Heizelement (14) steht.

7. Nicht brennbares Tabakrauchelement (60) nach Anspruch 6, wobei das tabakhaltige Element (79) an ein erstes Ende des äußeren Gehäuses angrenzt.

8. Nicht brennbares Tabakrauchelement (60) nach Anspruch 5, 6 oder 7, wobei sich das Heizelement in der Längsrichtung erstreckt.

9. Nicht brennbares Tabakrauchelement (60) nach einem der vorstehenden Ansprüche, weiter aufweisend:
ein Außenwandelement auf dem tabakhaltigen Element (79).

10. Nicht brennbares Tabakrauchelement (60) nach Anspruch 9, wobei das Außenwandelement einschließt,
einen äußeren Wandteil; und
einen inneren Wandteil, wobei der äußere Wandteil und das tabakhaltige Element (79) Abschnitte eines zweiten Luftkanals definieren.

11. Nicht brennbares Tabakrauchelement (60) nach Anspruch 10, wobei das Außenwandelement einschließt,
eine Abdeckung an einem ersten Ende des inneren Wandteils, wobei die Abdeckung den ersten Kanal abdeckt.

## Revendications

1. Élément de fumage de tabac non combustible (60) comprenant :
un élément de réservoir de la formulation pré-vapeur (22) configuré pour contenir un matériau de la formulation pré-vapeur ;
un élément de chauffage (14) couplé à l'élément de réservoir de la formulation pré-vapeur et configuré pour chauffer au moins une partie du matériau de la formulation pré-vapeur en une vapeur et fournir la vapeur à un premier canal ; et
un élément contenant du tabac (79) définissant au moins une partie du premier canal, l'élément contenant du tabac (79) chevauchant au moins une partie de l'élément de chauffage (14), où l'élément contenant du tabac (79) inclut une paroi intérieure (305) et une paroi extérieure (310), la paroi intérieure (305) étant perméable et la paroi extérieure (310) étant imperméable, où la paroi intérieure (305) et la paroi extérieure (310) contiennent du matériau de tabac végétal sous n'importe quelle forme.

2. L'élément de fumage de tabac non combustible (60) selon la revendication 1, dans lequel l'élément contenant du tabac (79) est un manchon annulaire.

3. L'élément de fumage de tabac non combustible (60) selon l'une quelconque des revendications précédentes, dans lequel l'élément de chauffage (14) est configuré pour être en communication thermique avec l'élément contenant du tabac (79) pour chauffer au moins une partie de l'élément contenant du tabac.

4. L'élément de fumage de tabac non combustible (60) selon la revendication 3, dans lequel l'élément de chauffage (14) est configuré pour chauffer la partie de l'élément contenant du tabac (79) et pour produire un arôme du tabac dans l'élément contenant du tabac.

5. L'élément de fumage de tabac non combustible (60) selon l'une quelconque des revendications précédentes, comprenant en outre :
une mèche (28) s'étendant dans une direction longitudinale depuis l'élément de réservoir de la formulation pré-vapeur (22).

6. L'élément de fumage de tabac non combustible (60) selon la revendication 5, dans lequel l'élément de réservoir de la formulation pré-vapeur (22) inclut,
un logement extérieur configuré pour contenir le matériau de la formulation pré-vapeur, un tube intérieur du logement extérieur définissant une entrée d'air, la mèche (28) étant en communication avec l'élément de réservoir de la formulation pré-vapeur (22) de telle sorte que la mèche (28) est configurée pour aspirer la partie du matériau de la formulation pré-vapeur pour être en communication thermique avec l'élément de chauffage (14).

7. L'élément de fumage de tabac non combustible (60) selon la revendication 6, dans lequel l'élément contenant du tabac (79) est adjacent à une première extrémité du logement extérieur.

8. L'élément de fumage de tabac non combustible (60) selon la revendication 5, 6 ou 7, dans lequel l'élément de chauffage s'étend dans la direction longitudinale.

9. L'élément de fumage de tabac non combustible (60) selon l'une quelconque des revendications précédentes, comprenant en outre :
un élément de paroi extérieure sur l'élément contenant du tabac (79).

10. L'élément de fumage de tabac non combustible (60) selon la revendication 9, dans lequel l'élément de paroi extérieure inclut,
une partie de paroi extérieure ; et
une partie de paroi intérieure, la partie de paroi extérieure et l'élément contenant du tabac (79) définissant des portions d'un deuxième canal d'air.

11. L'élément de fumage de tabac non combustible (60) selon la revendication 10, dans lequel l'élément de paroi extérieure inclut,
un couvercle à une première extrémité de la partie de paroi intérieure, le couvercle couvrant le premier canal.
